# EUROPÄISCHE PATENTANMELDUNG

(11) **EP 2 151 680 A1**
(43) Veröffentlichungstag der Anmeldung: **10.02.2010**
(21) Anmeldenummer: 08161816.7
(22) Anmeldetag: 05.08.2008
(51) Int. Cl.: G01N 21/86

(54) **Montageverfahren zur Herstellung eines Analysesystems**

(71) Anmelder: F. Hoffmann-La Roche AG, 4070 Basel (CH); Roche Diagnostics GmbH, 68305 Mannheim (DE)
(72) Erfinder: Miltner, Karl, 67227 Frankenthal (DE)
(74) Vertreter: Pfiz, Thomas

(57) **Zusammenfassung**

Die Erfindung betrifft ein Montageverfahren zur Herstellung eines Analysesystems, das eine Messeinheit (46) und eine Optikeinheit (52) zwischen der Messeinheit (46) und einem Vermessungsort aufweist, bei dem die Optikeinheit (52) relativ zu einer Lichtquelle (42) der Messeinheit (46) an einem Trägerteil (36) positioniert wird, wobei während der Positionierung die Strahlungsquelle (42) aktiviert wird und die Relativposition der Optikeinheit (52) und der Strahlungsquelle (42) mittels einer Positioniereinrichtung (20) variiert wird, bis elektromagnetische Strahlung von der Strahlungsquelle (42) auf den Vermessungsort auftrifft.

## Beschreibung

Die Erfindung betrifft ein Montageverfahren zur Herstellung eines Analysesystems, insbesondere eines tragbaren Blutzuckeranalysesystems, das eine vorzugsweise reflexionsphotometrische Messeinheit und eine Optikeinheit zwischen der Messeinheit und einem Vermessungsort aufweist, bei dem die Optikeinheit relativ zu einer elektromagnetischen Strahlungsquelle, insbesondere Lichtquelle der Messeinheit an einem Trägerteil positioniert wird, so dass der Vermessungsort mit elektromagnetischer Strahlung aus der Strahlungsquelle beaufschlagbar ist, wobei die Strahlungsquelle und die Optikeinheit in einer dauerhaften gegenseitigen Relativposition fixiert werden.

Blutzuckeranalysesysteme werden in Form von Handgeräten mit austauschbaren Testmitteln üblicherweise in Massenfertigung produziert, um Diabetikern die Möglichkeit zur täglich mehrfachen Selbstbestimmung ihres Blutzuckerspiegels zu geben. Zur Schmerzreduzierung bei der Blutgewinnung werden die Systeme zu einem geringeren Probenbedarf hin entwickelt. Dieser Trend verlangt bei den photometrischen Systemen immer kleinere Messflächen und damit verbunden miniaturisierte Messmodule in einem reduzierten Bauraum. Die an sich bekannten Messmodule weisen elektronische Bauteile und opto-mechanische Komponenten auf, die präzise zueinander angeordnet sein müssen, um den gewünschten Strahlengang zu verwirklichen. Zu diesem Zweck werden auf einem Trägerteil so genannte Passermarken aufgebracht, die als Positionsreferenz für die Montage der verschiedenen Komponenten dienen. Problematisch bei dieser Art der Positionierung ist, dass die Toleranzen der Fertigungswerkzeuge für die Komponenten voll in die optische Abbildung bei der Messung eingehen. Hinzu kommt der Einfluss von Montageabweichungen, insbesondere Bestückungstoleranzen, so dass unter Umständen ein hoher Prüfaufwand bzw. Ausschuss anfällt.

Ausgehend hiervon liegt der Erfindung die Aufgabe zugrunde, die im Stand der Technik bekannten Herstellungsverfahren weiter zu verbessern und einen wirtschaftlichen und dennoch präzisen Fertigungsprozess anzugeben.

Zur Lösung dieser Aufgabe werden die im Patentanspruch 1 angegebenen Montageschritte vorgeschlagen. Vorteilhafte Ausgestaltungen und Weiterbildungen der Erfindung ergeben sich aus den abhängigen Ansprüchen.

Die Erfindung geht von dem Gedanken aus, eine Feinjustage durch eine Simulation des Messprozesses im Fertigungsablauf zu ermöglichen. Dementsprechend wird erfindungsgemäß vorgeschlagen, dass während der Positionierung die Strahlungsquelle aktiviert wird und die Relativposition der Optikeinheit und der Strahlungsquelle mittels einer Positioniereinrichtung variiert wird, bis elektromagnetische Strahlung von der Strahlungsquelle auf den Vermessungsort auftrifft. Auf diese Weise wird die Positioniergenauigkeit nur durch die Leistungsfähigkeit der Positioniereinheit begrenzt, so dass die Toleranzkette erheblich reduziert werden kann.

Vorteilhafterweise wird die elektromagnetische Strahlung am Vermessungsort mit einem Kamerasystem als Leuchtfleck erfasst und die Relativposition zwischen Optikeinheit und Strahlungsquelle in Abhängigkeit von Bildparametern, insbesondere Größe, Position und Homogenität des erfassten Leuchtflecks variiert. Damit lässt sich ein für die Massenfertigung hinreichend schneller Montagezyklus mit einer besonders hohen Positioniergenauigkeit realisieren.

Eine weitere Verbesserung in der automatischen Fertigung wird dadurch erzielt, dass die Optikeinheit durch ein Handhabungsgerät der Positioniereinrichtung mit mindestens zwei, vorzugsweise drei translatorischen Freiheitsgraden in einem vorgegebenen Positionierbereich bewegt wird.

Vorteilhafterweise wird die elektromagnetische Strahlung über ein optisches Bauelement der Optikeinheit, insbesondere eine Linse, einen Lichtleiter oder eine Blende auf den Vermessungsort geführt. Die Simulation des Messvorgangs bei der Justage lässt sich dadurch realisieren, der Vermessungsort durch ein Blindelement anstelle eines für die Probenanalyse bestimmten analytischen Testelements definiert wird. Hierfür ist es für eine bildgebende Anordnung von Vorteil, wenn eine mattierte Glasplatte an dem Vermessungsort angeordnet und mit elektromagnetischer Strahlung beaufschlagt wird.

Eine weitere Fertigungsvereinfachung sieht vor, dass Komponenten der Messeinheit mittels eines leitfähigen Klebstoffs auf einer das Trägerteil bildenden Leiterplatte fixiert und zugleich elektrisch kontaktiert werden.

Um den Montagezyklus weiter zu verkürzen, ist es vorteilhaft, wenn die Optikeinheit durch mechanische Positioniermittel grob vorpositioniert wird.

Eine weitere günstige Verfahrensweise sieht vor, dass Klebepunkte aus einem vorzugsweise mit UV-Licht aushärtbaren Klebstoff für die Optikeinheit gebildet werden, und dass die Optikeinheit feinpositioniert und in der Endlage durch Aushärten der Klebepunkte dauerhaft mit der Messeinheit verbunden wird.

Für eine Massenfertigung mit kurzen Taktzeiten ist es vorteilhaft, wenn das Trägerteil in einem Transportprozess durch einen Bestückungsautomaten zum Aufbringen der Messeinheit und der Optikeinheit hindurchtransportiert wird.

Eine weitere Herstellungsvereinfachung lässt sich dadurch erreichen, dass die Optikeinheit als Spritzgussteil vorgefertigt wird.

Um einen standfesten Aufbau zu gewährleisten, ist es von Vorteil, wenn die Optikeinheit durch eine Vergussmasse feuchtigkeitsdicht mit der Messeinheit vergossen wird.

Im Folgenden wird die Erfindung anhand eines in der Zeichnung dargestellten Ausführungsbeispiels näher erläutert. Es zeigen:
- Fig. 1: eine Montagestation für ein Optikmodul eines Blutzuckermessgeräts in der Draufsicht;
- Fig. 2: die Komponenten des Optikmoduls in perspektivischer Ansicht.

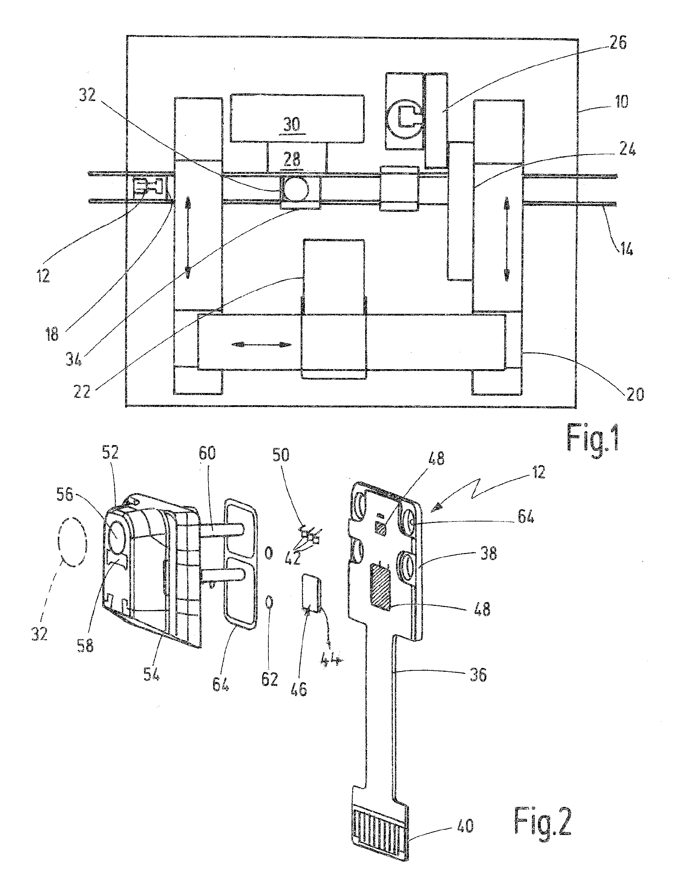

Fig. 1 zeigt eine Montagestation 10 zur automatischen Fertigung eines Optikmoduls 12 eines tragbaren Blutzuckermessgeräts. Das fertige Blutzuckermessgerät bildet zusammen mit analytischen Verbrauchsmitteln in Form von Testbändern oder Teststreifen ein analytisches System, das vor Ort vom Anwender selbst für Blutzuckermessungen einsetzbar ist. Dabei werden trockenchemische Testelemente bzw. Testfelder auf dem analytischen Verbrauchsmittel mit einer Blutprobe beaufschlagt und mittels des Optikmoduls 12 reflexionsphotometrisch ausgewertet. Die Testfelder definieren somit einen Probenvermessungsort, auf den das Optikmodul 12 mit möglichst geringen Fertigungstoleranzen lagegenau ausgerichtet sein soll. Einzelheiten eines solchen Analysesystems ergeben sich beispielsweise aus der EP-A 1 760 469, auf die in diesem Zusammenhang Bezug genommen wird.

Die in Fig. 1 dargestellte Montagestation 10 umfasst ein Transportband 14 zur Durchführung von Werkstückträgern 18, auf denen das Optikmodul 12 aufgebaut wird. Das Transportband läuft durch eine Positioniereinrichtung 20, die eine in einer X-Y-Ebene bewegliche Kinematik aufweist. Daran ist ein zusätzlich in Z-Richtung beweglicher Handhabungskopf 22 zum Aufnehmen und 3D-Positionieren von Bauteilen angeordnet. Eingangsseitig sind an dem Transportband zwei Klebstoff-Applikationsgeräte 24, 26 angeordnet. Über dem Positionierbereich des Handhabungskopfes 22 befindet sich ein Kamerasystem 28, das mittels der Steuereinrichtung 30 eine kamerageführte Bewegungssteuerung des Handhabungskopfes 22 erlaubt. Eine mattierte Glasplatte 32 bildet dabei eine Bildebene als Vermessungsort, der mit der vorgesehenen Lage eines Testfelds (dem Probenvermessungsort) beim Einsatz des Analysegeräts korrespondiert. Weiterhin ist zur Aushärtung von Klebestellen eine UV-Belichtungseinheit 34 vorgesehen.

Wie aus Fig. 2 ersichtlich, umfasst das Optikmodul 12 eine Trägerplatine 36, die im Kopfbereich mittels Rückenplatte 38 verstärkt ist und ein Steckerende 40 mit elektrischen Steckkontakten besitzt. Auf der Platine 36 sind drei Leuchtdioden als Lichtquelle 42 und eine Photodiode als Lichtempfänger 44 einer reflektometrischen Messeinheit 46 kontaktierbar. Dies erfolgt einerseits über elektrische Leiterflächen 48 in einem Klebeprozess und andererseits über separate Anschlussdrähte 50 in einem so genannten "Drahtbondprozess".

Als weiteres Bauelement umfasst das Optikmodul 12 eine Optikeinheit 52, die den Strahlengang zwischen der Messeinheit 46 und dem Vermessungsort 32 bestimmt. Die Optikeinheit 52 weist ein Spritzgussgehäuse 54 auf, in das eine Linse 56 in Zweikomponenten-Spritzgusstechnik eingeformt ist. Die Linse 56 bündelt das Licht der Lichtquelle 42 auf den Vermessungsort 32, während eine Blende 58 für das zurückgeworfene Licht dem Detektor 44 vorgeordnet ist.

Für die Verbindung der Optikeinheit 52 sind Positionierstifte 60 sowie Klebepunkte 62 vorgesehen, während eine flexible Abschirmung 64 das Eindringen von Fremdlicht verhindert.

Zur Herstellung von Optikmodulen 12 werden Werkstückträger 18 in einem getakteten Montageprozess durch die Montagestation 10 transportiert. Dabei wird zunächst ein elektrisch leitender Kontaktkleber durch das Applikationsgerät 26 auf den Leiterflächen 48 aufgestempelt, um die LEDs 42 und die Photodiode 44 fest aufzukleben. Das zweite Applikationsgerät 24 dient zum Aufbringen der Klebepunkte 62 für die Optikeinheit 52. Diese wird nachfolgend durch den Handhabungskopf 22 aufgesetzt, wobei eine grobe Vorpositionierung durch die Positionierstifte 60 in den ein Übermaß aufweisenden Platinenbohrungen 64 erfolgt.

Für die anschließende Feinpositionierung wird die Lichtquelle 42 aktiviert bzw. unter Spannung gesetzt, so dass in Abhängigkeit von der Momentanposition der Optikeinheit 52 ein Leuchtfleck auf der Glasplatte 32 erscheint. Die Position, Größe und Homogenität des Leuchtflecks wird während der Positionsveränderung durch die Kamera 28 erfasst und in der Steuereinrichtung 30 mit einem vorgegebenen Signalmuster für eine Sollposition verglichen. Dabei wird die Position der Optikeinheit 52 vornehmlich in der X-Y-Ebene in einem Toleranzkreis von etwa 5 µm Radius variiert, wobei aber auch senkrecht dazu ein Toleranzausgleich in Z-Richtung möglich ist. Sodann werden in der ausgerichteten Endlage die Klebepunkte 62 mittels der UV-Lichtquelle 34 ausgehärtet, um die gegenseitige Relativposition von Messeinheit 46 und Optikeinheit 52 dauerhaft zu fixieren. Zusätzlich kann eine Vergussmasse zur Abdichtung aufgebracht werden. Das Justageergebnis kann mittels des Kamerasystems 28, 30 abschließend überprüft werden, bevor das fertige Optikmodul 12 aus der Station 10 ausgeschleust wird.

Im weiteren Fertigungsprozess des Analysegeräts wird das Optikmodul 12 auf eine Hauptplatine aufgesteckt, die in einem Gerätegehäuse angeordnet ist (nicht gezeigt). Die Verbindung kann mittels der freien Enden der Positionierstifte 60 hergestellt werden, die durch so genanntes Warmverstemmen fest fixiert werden. Dabei wird eine definierte Lagebeziehung zu einem Testelement sichergestellt, das sich an einem Probenvermessungsort im gleichen Abstand wie zuvor die Glasplatte 32 vor dem Optikmodul 12 befindet.

## Patentansprüche

1. Montageverfahren zur Herstellung eines Analysesystems, insbesondere eines tragbaren Blutzuckeranalysesystems, das eine vorzugsweise reflexionsphotometrische Messeinheit (46) und eine Optikeinheit (52) zwischen der Messeinheit (46) und einem Vermessungsort aufweist, umfassend:
a) Positionieren der Optikeinheit (52) relativ zu einer elektromagnetischen Strahlungsquelle (42), insbesondere Lichtquelle der Messeinheit (46) an einem Trägerteil (36), so dass der Vermessungsort mit elektromagnetischer Strahlung aus der Strahlungsquelle (42) beaufschlagbar ist,
b) Fixieren der Strahlungsquelle (42) und der Optikeinheit (52) in einer dauerhaften gegenseitigen Relativposition,
**dadurch gekennzeichnet, dass**
c) während der Positionierung die Strahlungsquelle (42) aktiviert wird und die Relativposition der Optikeinheit (52) und der Strahlungsquelle (42) mittels einer Positioniereinrichtung (20) variiert wird, bis elektromagnetische Strahlung von der Strahlungsquelle (42) auf den Vermessungsort auftrifft.

2. Montageverfahren nach Anspruch 1, bei dem die elektromagnetische Strahlung am Vermessungsort mit einem Kamerasystem (28) als Leuchtfleck erfasst wird, und bei dem die Relativposition zwischen Optikeinheit (52) und Strahlungsquelle (42) in Abhängigkeit von Bildparametern, insbesondere Größe, Position und Homogenität des erfassten Leuchtflecks variiert wird.

3. Montageverfahren nach Anspruch 1 oder 2, bei dem die Optikeinheit (52) durch ein Handhabungsgerät (22) mit mindestens zwei, vorzugsweise drei translatorischen Freiheitsgraden in einem Positionierbereich bewegt wird.

4. Montageverfahren nach einem der Ansprüche 1 bis 3, bei dem die elektromagnetische Strahlung über ein optisches Bauelement (56,58) der Optikeinheit (52), insbesondere eine Linse, einen Lichtleiter oder eine Blende geführt wird.

5. Montageverfahren nach einem der Ansprüche 1 bis 4, bei dem der Vermessungsort durch ein Blindelement (32) anstelle eines für die Probenanalyse bestimmten analytischen Testelements definiert wird.

6. Montageverfahren nach einem der Ansprüche 1 bis 5, bei dem eine mattierte Glasplatte an dem Vermessungsort angeordnet und mit elektromagnetischer Strahlung beaufschlagt wird.

7. Montageverfahren nach einem der Ansprüche 1 bis 6, bei dem Komponenten (42,44) der Messeinheit (46) mittels eines leitfähigen Klebstoffs auf einer das Trägerteil (36) bildenden Leiterplatte fixiert und dabei elektrisch kontaktiert werden.

8. Montageverfahren nach einem der Ansprüche 1 bis 7, bei dem die Optikeinheit (52) durch mechanische Positioniermittel (60) grob vorpositioniert wird.

9. Montageverfahren nach einem der Ansprüche 1 bis 8, bei dem Klebepunkte (62) aus einem vorzugsweise mit UV-Licht aushärtbaren Klebstoff für die Optikeinheit (52) gebildet werden, und bei dem die Optikeinheit (52) feinpositioniert und in der Endlage durch Aushärten der Klebepunkte (62) fest mit der Messeinheit (46) verbunden wird.

10. Montageverfahren nach einem der Ansprüche 1 bis 9, bei dem das Trägerteil (36) in einem Transportprozess durch einen Bestückungsautomaten (10) zum Aufbringen der Messeinheit (46) und der Optikeinheit (52) hindurchtransportiert wird.

11. Montageverfahren nach einem der Ansprüche 1 bis 10, bei dem die Optikeinheit (52) als Spritzgussteil vorzugsweise einstückig vorgefertigt wird.

12. Montageverfahren nach einem der Ansprüche 1 bis 11, bei dem die Optikeinheit (52) durch eine Vergussmasse feuchtigkeitsdicht mit der Messeinheit (46) vergossen wird.
